Europäisches Patentamt

European Patent Office

Office européen des brevets

(11) Publication number: 0 370 023 B1

# EUROPEAN PATENT SPECIFICATION

(45) Date of publication of patent specification: 09.11.94 (51) Int. Cl.5: C12P 7/06

(21) Application number: 88904580.3

(22) Date of filing: 24.05.88

(86) International application number:
PCT/GB88/00411

(87) International publication number:
WO 88/09379 (01.12.88 88/26)

The file contains technical information submitted after the application was filed and not included in this specification

(54) THERMOPHILIC ETHANOL PRODUCTION.

(30) Priority: 27.05.87 GB 8712410

(43) Date of publication of application:
30.05.90 Bulletin 90/22

(45) Publication of the grant of the patent:
09.11.94 Bulletin 94/45

(84) Designated Contracting States:
AT BE CH DE FR GB IT LI NL SE

(56) References cited:

BIOTECH 83, 04-06 May 1983, Lomdon (GB), Online Publications Ltd., Northwood (GB); B.S. HARTLEY et al., pp. 895-905&NUM;

PHILOSOPHICAL TRANSACTIONS OF THE ROYAL SOCIETY OF LONDON, A., Mathematical & Physical Sciencesm vol. 321, no. 1561, 30 April 1987, The Royal Society, London (GB); B.S. HARTLEY et al., pp. 555-568&NUM;

(73) Proprietor: ELSWORTH BIOTECHNOLOGY
LIMITED
Grove Cottage, Smith Street, Elsworth
Cambridge CB3 8HY (GB)

(72) Inventor: HARTLEY, Brian, Selby
Grove Cottage
Smith Street
Elsworth Cambridge CB3 8HY (GB)

(74) Representative: Farwell, William Robert et al
PHILLIPS & LEIGH
7 Staple Inn
High Holborn
London WC1V 7OF (GB)

TRENDS IN BIOTECHNOLOGY, vol. 2, no. 6, 1984, Elsevier Science Publishers BV, Amsterdam (NL); M.A. PAYTON, pp. 153-158&NUM;

FEMS Microbiology Letters, vol. 26, 1985, Elsevier; M.A. PAYTON et al., pp. 333-336&NUM;

**Description**

FIELD OF INVENTION

The invention relates to alcohol, that is to say ethanol, production by fermentation.

GENERAL DISCUSSION

Alcohol production from waste or by-product sugars whether arising as such or derived from conversion of other carbohydrates has long been known but is currently of growing importance. Cheap oil at present, and severe food shortages in particular regions, cannot detract from the basic unsoundness of relying on non-renewable energy sources when, properly managed, agriculture could provide food and energy world wide.

We have studied known alcohol production processes, largely by yeasts, and concluded that a key improvement in economic operation, if achievable in practice, is use of temperatures at which the alcohol can conveniently be removed directly as vapour from the fermentation medium. Yeasts of course are incapable of growth at such temperatures, and we have turned to thermophilic bacteria.

Yeasts ferment only glucose, maltose or sucrose whereas some bacteria can also utilise cellobiose from enzymic hydrolysis of cellulose or xylose and arabinose from hydrolysis of hemicellulose. The latter (pentose) sugars are the major components of waste streams from paper-making or from pretreatments of straw such as steam-explosion or dilute acid hydrolysis. The economics of ethanol production from sugar cane would for example be greatly improved if the bagasse could be so utilised as well as the juice.

Some thermophiles have been described which can utilise all these sugars to produce high yields of ethanol e.g. Clostridium thermosaccharolyticum, Cl. thermohydrosulfuricum or Thermoanaerobacter ethanolicus. However they are strict anaerobes and their reported properties compare unfavourably with the Bacillus stearothermophilus strains described below. Moreover we have seen that facultative anaerobes have additional advantages by allowing a novel mixed aerobic-anaerobic process which allows by-products of the anaerobic phase to be utilised aerobically to regenerate catalytic biomass.

Most facultative anaerobes do not produce high yields of ethanol. In previous disclosures we described a 'metabolic steering' strategy whereby mutants believed to be of Bacillus stearothermophilus NCA 1503 may be manipulated to make high yields of ethanol [1, 2]. That strategy involved eliminating L-lactate production by selecting mutations in L-lactate dehydrogenase. The resulting mutant was expected to make acetate, ethanol and formate anaerobically in yields of 2:2:4 per mole of sucrose. Surprisingly however yields of ethanol were higher than this theoretical maximum under certain conditions, notably low pH and higher temperatures, and this was ascribed to a catalytic conversion of sucrose to ethanol + $CO_2$ during a final non-growth stage in batch cultures.

We have now discovered that the results previously reported are to an extent in error in that the organism described is not a derivative of B. stearothermophilus NCA 1503 (NCIB 8924) as we assumed (Payton and Hartley [3]) nor indeed of any specific known thermophilic bacillus. Instead it appears to be derived from a novel strain of the species Bacillus stearothermophilus that has properties that make it much superior to known strains for the purposes described above. In particular, it has a much higher growth rate than strain NCA 1503 both aerobically and anaerobically at temperatures above 60°C and grows anaerobically above 70°C, at which temperature growth ceases with strain NCA 1503. Moreover, it utilises both cellobiose and the pentose sugars found in a crude dilute acid hydrolysis of wheat straw produced by the ICI process described by Ragg and Fields [4].

Hence though this invention is not restricted to particular bacteria, it concerns facultative anaerobes such as B. stearothermophilus strain LLD-R (NCIB deposit details below) that rapidly ferment a wide range of sugars including cellobiose and pentoses both aerobically and anaerobically above 70°C. Such strains would normally produce lactate anaerobically, but this pathway is eliminated by selecting mutations in NAD-linked lactate dehydrogenase.

The invention broadly is defined as production of ethanol by a process in which a strain of Bacillus stearothermophilus or other thermophilic, facultatively anaerobic bacterium is selected for the characteristics of fermenting sugars both aerobically and anaerobically and of being active in anaerobic fermentation at 70°C or above and

(i) anaerobic fermentation is carried out with continuing removal of ethanol at 70°C or above

(ii) the fermentative activity of the bacterium is maintained by withdrawing a proportion of the anaerobic fermentation medium on a continuing basis preferably with removal of ethanol and allowing the bacteria therein to multiply aerobically, using residual sugars or metabolites thereof present in the medium,

3

before being returned to the anaerobic fermentation.

Desirably the bacterium is selected for the further characteristic of entering, after an aerobic or anaerobic growth phase, a non-multiplying but metabolically active anaerobic phase wherein essentially only ethanol is produced, and the anaerobic fermentation is carried out substantially in that phase.

Further acetate production may be suppressed by physiological controls such as acid pH, higher temperatures or high levels of extracellular acetate, or by further genetic lesions in enzymes of the acetate pathway. This leads to a channelling of anaerobic metabolism via pyruvate dehydrogenase, resulting in conversion of sugars to ethanol + $CO_2$. The resulting cells may not grow anaerobically but can catalyse conversion of sugars to ethanol without growth.

Hence an aspect of this invention is a process in which such cells are used in a catalytic anaerobic production stage fed with sugars but minimising growth. Most of the ethanol is automatically removed into the vapour phase above 70°C, so the production phase can be fed with high sugar concentrations without exceeding the ethanol tolerance of the organism (ca. 4% w/v). We have seen that these properties lend themselves to a novel continous process in which the optimum fermenter productivity is achieved by continous cell recycle after removing aqueous phase products by centrifugation or filtration. The minimum growth rate necessary to maintain catalytic viability can be achieved by bleeding off a small proportion of cells during recycle; this results in conversion of an equivalent proportion of the influent sugar into fresh biomass. Alternatively, the remaining aqueous phase ethanol is removed before returning spent cells, unhydrolysed sugars, residual traces of ethanol and by-products such as acetate and formate to an aerobic 'biomass' stage. The aerobic cells are then returned to the catalytic "production" stage, if necessary through an intervening anaerobic "adaptation" stage. An attractive feature of such a reactor conformation is that automatic process control to optimise ethanol yield can be maintained by minimising aerobic $CO_2$ and maximising anaerobic $CO_2$.

Specific advantageous features may be:-

i) Use of process conditions adjusted so as to maximise the ratio of anaerobically to aerobically produced $CO_2$, thus maximising ethanol yield.

ii) Use of bacteria specifically selected as lacking NAD-linked lactate dehydrogenase activity.

iii) Use of bacteria specifically selected as producing ethanol essentially by pyruvate dehydrogenase pathway activity, and in particular bacteria in which, during the fermentation, pyruvate-formate lyase pathway flux is suppressed by reason of mutation or by conditions causing intracellular accumulation of metabolites.

## HISTORY AND CHARACTERISTICS OF STRAINS

Bacillus stearothermophilus strain LLD-15 (NCIB deposit details below) arose during attempts to obtain mutants of Bacillus stearothermophilus strain NCA 1503 lacking L-lactate dehydrogenase activity by selecting for suicide substrate resistance (Payton and Hartley [3]). It was naturally assumed to be a mutant of the latter strain, but in fact is believed derived from a novel extreme thermophile, Bacillus stearothermophilus strain LLD-R. Strain LLD-R arises spontaneously and reproducibly from strain LLD-15 and is selected on plates or during continuous cultures under which it grows more rapidly, e.g. at low pH in media containing sugars + acetate and formate. It produces L-lactate anaerobically and contains high levels of L-lactate dehydrogenase so it is clearly a 'wild-type' revertant of the LLD-15 lesion.

Both the mutant strain B. stearothermophilus strain LLD15 and the wild-type, strain LLD-R, are gram positive, spore-forming rods that resemble the broad class Bacillus stearothermophilus in morphology and growth temperature range. However in a series of biochemical and growth tests (Table 1) they differ from B. stearothermophilus NCA 1503 and all other related strains in the extensive collection of Sharp et. al. J. Gen. Microbiol. 117 201 (1980). These properties merit a general classification as Bacillus stearothermophilus following Donk, L. (1920) J. Bacteriol. 5 373, but the growth temperature range is distinctly higher than that for strain NCA 1503, from which the mutant was thought to derive. Hence both organisms are deposited as novel type strains Bacillus stearothermophilus LLD-R (NCIB 12403) and Bacillus stearothermophilus LLD-15 (NCIB 12428) with the National Collection of Industrial and Marine Bacteria, Torry Research Station, P.O. Box 31, Aberdeen, AB9 8DG, Scotland. The respective dates of deposit are 10th February 1987 and 9th April 1987.

Strains LLD-R and LLD-15 grow well on a rich BST medium (g/l): tryptone (Oxoid) 20.0; yeast extract (Oxoid) 10.0; $K_2SO_4$, 1.3; $MgSO_4.7H_2O$, 0.27; $MnCl_2.4H_2O$, 0.015; $FeCl_3.6H_2O$, 0.007; citric acid, 0.32; supplemented with an appropriate carbon source and adjusted to the required pH with KOH or $H_2SO_4$. However we have also developed a fully defined Medium BST-MM (g/l): C source variable; $K_2SO_4$, 0.3; $Na_2HPO_4$, 1.0; $MgSO_4$, 0.4; $MnCl_2.4H_2O$, 0.003; $CaCl_2$, 0.005; $NH_4Cl$, 1.0; citric acid, 0.16; methionine, 0.2;

(mg/l): nicotinic acid, 10; biotin, 10; thiamine, 10; $ZnSO_4.7H_2O$, 0.4; boric acid, 0.01; $CoCl_2.6H_2O$, 0.05; $CuSO_4.5H_2O$, 0.2; $NiCl_3.6H_2O$, 0.01; EDTA, 0.25.

TABLE 1.   Comparison of new strains with other thermophilic bacilli

| | B. stearo. LLD-15 | B. stearo. LLD-R | B. stearo. NCA 1503 | B. stearo. ATCC 12016 | B. stearo. 240 | B. stearo. 262 | B. stearo. RS93 | B. stearo. 136 | B. stearo. 10 | B. caldotenax | B. caldovelox | B. caldolyticus | B. coagulans ATCC 8083 | B. coagulans ATCC 12245 |
|---|---|---|---|---|---|---|---|---|---|---|---|---|---|---|
| Starch hydrolysis | R | .. | + | + | R | R | − | − | + | R | R | + | − | − |
| Casein hydrolysis | + | .. | + | w | + | + | − | − | + | + | + | + | − | w |
| Gelatin hydrolysis | + | .. | + | w | − | + | − | w | + | + | + | + | − | − |
| Hippurate hydrolysis | + | .. | − | .. | .. | .. | .. | .. | .. | + | .. | .. | .. | .. |
| Citrate utilisation | − | − | − | .. | .. | .. | .. | .. | .. | + | .. | .. | .. | .. |
| Catalase | + | + | − | − | + | + | + | + | − | − | − | − | + | + |
| Oxidase | − | − | − | + | + | + | + | + | − | + | + | + | − | − |
| Growth in 3% saline | − | .. | w | − | w | + | + | w | w | w | − | − | − | − |
| Sugar fermentations: | | | | | | | | | | | | | | |
| Galactose | − | − | w | + | − | − | − | − | w | − | − | − | + | + |
| Glycogen | − | .. | + | w | + | + | − | − | + | w | − | + | w | − |
| Mannitol | − | − | − | w | − | − | + | + | − | w | + | w | w | − |
| Raffinose | − | − | + | − | − | − | − | − | + | + | w | − | − | − |
| Starch | − | .. | + | − | + | w | − | − | + | − | − | + | w | w |
| Trehalose | + | .. | + | − | + | − | w | w | + | w | + | + | + | + |
| Xylose | + | + | − | + | − | − | − | − | + | − | − | − | w | w |

Test were performed in parallel according to Sharp, R.J.,
Bown, K.J. and Atkinson R. (1980) on B. stearothermophilus strain
LLD-15, LLD-R and NCA 1503 and B. caldotenax.   The other data
are from that reference; R, restricted; +, positive; w, weak positive;
−, negative; .., not tested.

LIST OF FIGURES

The text refers to Figs. 1 - 6 which are as follows:-
Fig. 1     Anaerobic pathways and theoretical yields in Bacillus stearothermophilus strain LLD-15
Fig. 2     Steady state values in continuous cultures at pH7, D = O.2hr$^{-1}$ on sucrose 10g/l anaerobically
          or 5g/l aerobically (dotted line), 0.5% tryptone, 0.25% yeast extract

5

a) Biomass: (●) strain NCA 1503, (■) strain LLD-R, (O) strain LLD-15

b) Products with strain LLD-15

Fig.3    Continuous cultures of strain LLD-15 on various concentrations of wheat-straw hydrolysate (Ragg and Fields, 1986) at 70°C, D = 0.2hr$^{-1}$, pH7.

Fig. 4    A continuous two-stage reactor system for ethanol production.

Fig. 5    Continuous fermentation with partial cell recycle, plant.

Fig. 6    Continuous fermentation with partial cell recycle, relationships.

ANAEROBIC PATHWAYS IN THE NEW STRAINS

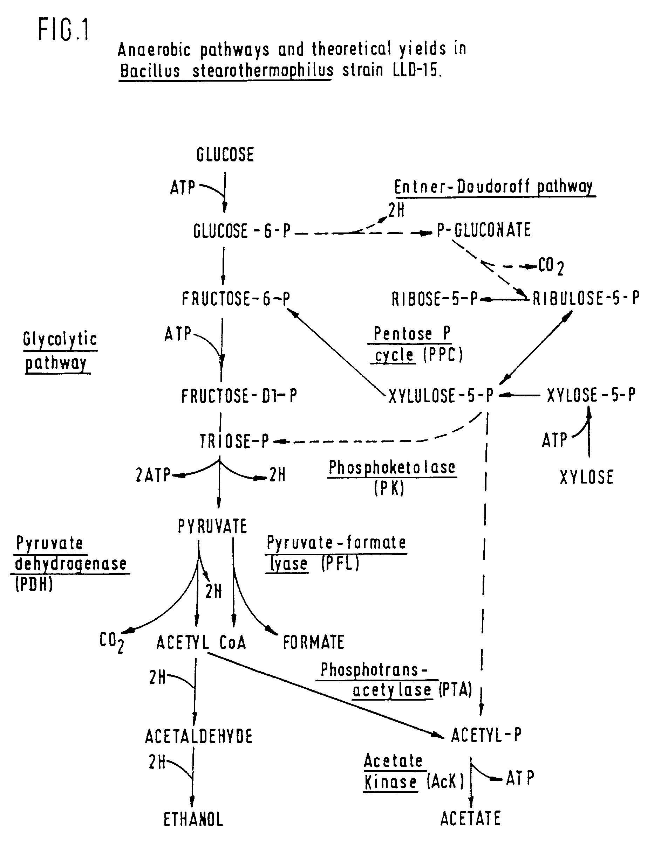

Our original experiments were conducted mostly at 60°C in batch culture on 2.35% (w/v) sucrose/BST medium since that is the optimal temperature for strain NCA 1503 which was the presumed ancestor of the mutant strain LSD-15. In anaerobic batch cultures of strain NCA 1503 or strain LLD-R the final product is predominantly L-lactate, whereas strain LSD-15 gives (moles/mole sucrose): ethanol (1.8), acetate (1.8) and formate (3.2) at pH 7.9. This is consistent with metabolism via the pyruvate-formate lyase (PFL) pathway (Fig. 1) since the mutation abolishes L-lactate dehydrogenase activity. However at more acid pH the ratio of ethanol to acetate increases becoming at pH 6.2: ethanol (2.9), acetate (0.2) and formate (1.3). This indicates that a novel pathway leading to 2 ethanol + 2 $CO_2$ from each glucose residue is also operating, and we believe that this is via pyruvate dehydrogenase (PDH) which is generally considered to be inoperative anaerobically. In summary the products in Fig. 1 are:-

| Pathway | Products (moles/mole sugar) | | | | |
|---|---|---|---|---|---|
| | Ethanol | Acetate | Formate | $CO_2$ | ATP |
| **Glucose:** | | | | | |
| Glycolysis + PDH | 2.00 | 0.00 | 0.00 | 2.00 | 2.00 |
| " + PFL | 1.00 | 1.00 | 2.00 | 0.00 | 3.00 |
| Entner-Doudoroff + PDH | 1.50 | 0.50 | 0.00 | 0.00 | 3.00 |
| " " + PFL | 1.00 | 1.00 | 1.00 | 1.00 | 2.00 |
| **Xylose:** | | | | | |
| Pentose cycle + PDH | 1.67 | 0.00 | 0.00 | 1.67 | 1.67 |
| " " + PFL | 0.83 | 0.83 | 1.67 | 0.00 | 2.50 |
| Phosphoketolase + PDH | 1.00 | 1.00 | 0.00 | 1.00 | 1.50 |
| " + PFL | 0.50 | 1.50 | 1.00 | 0.00 | 2.50 |

The switch from the PFL- to the PDH-pathway occurs in the later stages of anaerobic batch fermentations with strain LLD-15 and is associated with a slowing in growth rate and the appearance of traces of pyruvate in the medium. The effect is most marked during batch fermentations on high sugars, e.g. 5% (w/v) sucrose, where growth ceases long before all the sugar is utilised but the non-growing cells continue to convert sucrose quantitatively to ethanol + $CO_2$. Hence in such a batch fermentation ethanol yields can reach 3.64 moles/mole sucrose (91% theoretical). Higher temperature (70°C) also favours a switch to the PDH-pathway.

The switch from the PFL to the PDH pathway is due to accumulation of acetate and formate in the medium, and not due to ethanol accumulation, as shown by adding these products to the medium in the early stages of a batch fermentation. Pyruvate secretion is observed whenever the PDH-pathway is significantly operative. Cells grown under such conditions show levels of pyruvate dehydrogenase activity in cell-free extracts even higher than those in fully aerobic cells, whereas the wild-type shows very low anaerobic PDH levels. There is no detectable pyruvate decarboxylase or formate dehydrogenase activity, which might have provided an alternative pathway for ethanol and $CO_2$ production. The switch in pathways may be a pre-sporulation phenomenon as biomass decreases towards the end of batch fermentations and spores are observed.

6

The probable reason for the switch in pathways is as follows. The wild type organism is geared to rapid growth, both aerobic and anaerobic, so has rapidly acting sugar uptake and glycolysis systems. It normally secretes L-lactate under anaerobic conditions, but when that pathway is blocked pyruvate metabolism is shunted into the PFL-pathway. However the rate of excretion of acetate and formate then becomes the rate-limiting step for energy metabolism, particularly in the presence of external acetate and formate or at acid pH where secretion against an anion or proton gradient reduces the efflux rate. Hence pyruvate accumulates within the cell and induces the pyruvate dehydrogenase activity to levels even higher than in fully aerobic cells. The flux through pyruvate dehydrogenase is however still inadequate to maintain the rapid growth rate seen at alkaline pH or at low sugar concentration in the absence of acetate and formate, and the cells reach a stationary stage in which sugars are converted quantitatively to ethanol and $CO_2$ without growth.

SINGLE STATE CONTINUOUS CULTURES

Preliminary experiments to compare wild type (strain LLD-R) and strain LLD-15 were conducted with 2-3% sucrose in BST medium at 60°C (dilution rate 0.25 $hr^{-1}$). As expected, wild type cells produced predominantly L-lactate, ranging from 3.13 moles/mole sucrose consumed at pH8 to 3.50 at pH 6.35 and Y values (g.cells/g.sucrose) were around 0.07. With the mutant strain at pH7 ethanol was the major product (2.3 moles/mole sucrose) and the Y value was higher (0.10). However strain LLD-15 was unstable in continuous cultures at acid pH or at high sugar concentrations, and takeover by revertants to L-lactate production (strain LLD-R) was common. This reflects the powerful selection pressure for increased energy efficiency exerted by such continuous cultures, and illustrates a potential defect of a continuous process. However this reversion is less frequent in continuous cultures at 70°C on lower sugar concentrations and can be eliminated by reselection from strain LLD-R of a non-reverting mutant (procedure of Payton and Hartley[3]).

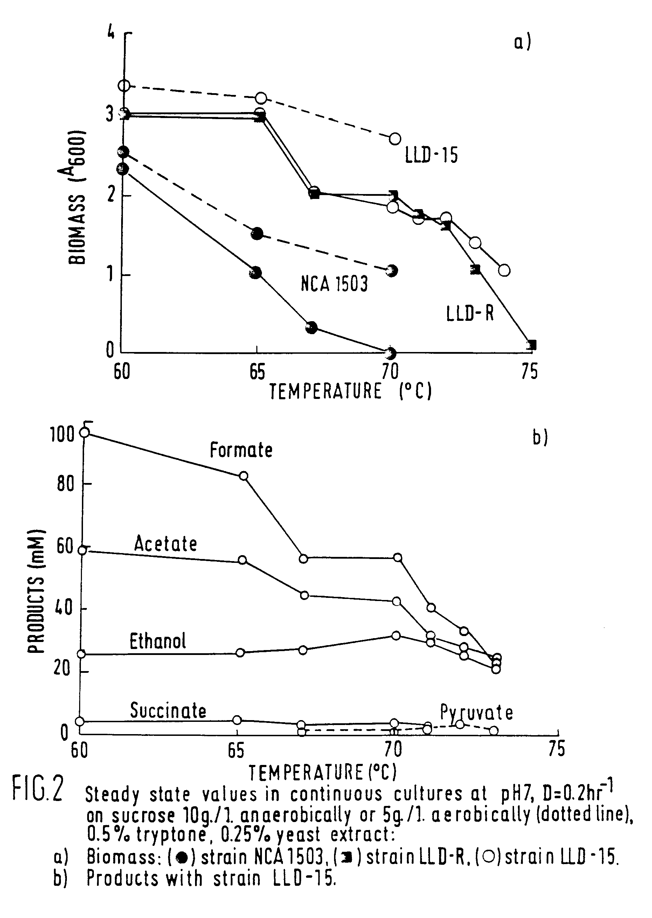

Fig. 2a shows the steady-state biomass in continuous cultures of strains NCA 1503, LLD-R and LLD-15 grown anaerobically on 1% (w/v) sucrose or aerobically on 0.5% sucrose, 0.5% tryptone, 0.25% yeast extract at pH7.0, dilution rate 0.2 $hr^{-1}$, at various temperatures. Both the new strains and strain NCA 1503 show efficient aerobic and anaerobic metabolism, but strain NCA 1503 expires anaerobically above 70°C whereas both the wild-type (LLD-R) and the mutant (LLD-15) retain significant anaerobic metabolism up to 75°C. This temperature is closer to the boiling point of aqueous ethanol and is therefore significant for the processes described herein.

The products from the anaerobic continuous cultures of strain LLD-15 are shown in Fig. 2b. It is clear that ethanol productivity (mmol/$A_{600}$) rises as temperature increases and is associated with pyruvate secretion.

Strain NCA 1503 does not grow on xylose either aerobically or anaerobically, but both LLD-R and LLD-15 do. The results with sucrose can be compared with continuous cultures on 1% xylose at 70°C in analogous conditions (Table 2). Steady states can again be maintained and alcohol yields are again higher at acid pH.

7

TABLE 2. Continuous anaerobic cultures of strain LLD-15 on xylose (10 g/l), tryptone (5 g/l), yeast extract (2.5 g/l), BST salts at 70$^{\circ}$C, D = 0.2 hr$^{-1}$

| | | | Products: | | |
|---|---|---|---|---|---|
| pH | Cells | Residual Xylose | (moles/mole | xylose, g/g | cells/hr) |
| | (g/l) | (g.l) | Ethanol | Acetate | Formate |
| 6.5 | 0.35 | 0.9 | 1.70 (0.13) | 1.79 (0.17) | 3.48 (0.25) |
| 7.0 | 0.98 | 0.9 | 1.43 (0.04) | 2.14 (0.07) | 3.84 (0.10) |
| 8.0 | 0.67 | 0.9 | 0.82 (0.03) | 1.07 (0.05) | 2.20 (0.08) |

The steady state biomass at pH7 is less than that with half the concentration of sucrose so xylose metabolism is less energy efficient. Sucrose is believed to be metabolised to two hexose phosphates via a relevant kinase + phosphorylase, requiring one ATP. In contrast two ATP's are believed necessary to product two pentose phosphate molecules. Hence sucrose is intrinsically a better energy substrate.

The products of the continuous xylose fermentations show that an appreciable proportion of the tryptone-yeast extract is metabolised for energy production. Nevertheless the product ratios at pH8 indicate that metabolism proceeds via the pentose phosphate pathway, glycolysis and the PFL-pathway. The ethanol yields are lower than on sucrose, suggesting little flux through the PDH-pathway. However fermentations on higher sugar concentrations at higher temperatures and lower pH are expected to increase the flux through the latter pathway, as for sucrose, and mutations in acetate kinase or phosphotransacetylase to produce similarly, a non-growing strain that converts xylose quantitatively to ethanol and $CO_2$.

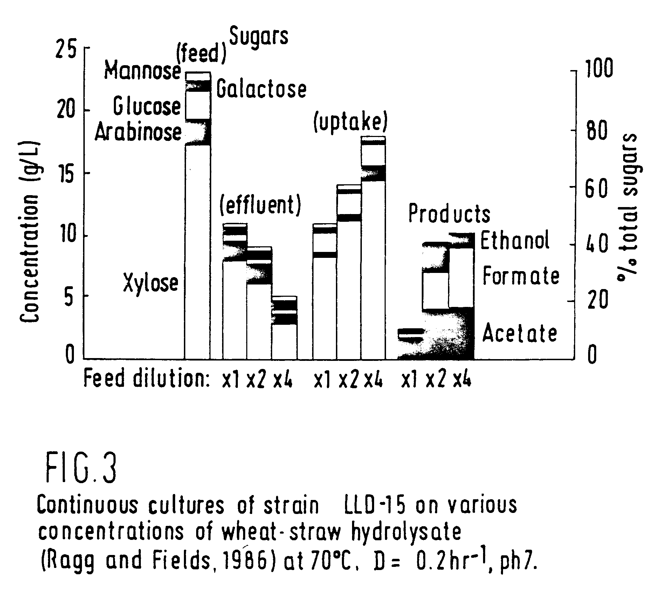

Hence the new strain and derivatives thereof are an organism of choice for ethanol production from hydrolysates of lignocellulosic wastes. Continuous cultures have been performed on crude hydrolysates of wheat straw produced by the ICI hydrolysis process described by Ragg and Fields[4] . This is effectively a waste stream, rich in xylose and lignin, produced by a short dilute acid hydrolysis step designed to remove hemicelluloses and thereby facilitate subsequent delignification. The crude material is adjusted to pH7.0 and tested in continuous culture with the new strain at 70$^{\circ}$C, D = 0.2 hr$^{-1}$ at various dilutions as shown in Fig. 3. The waste-stream provides all necessary nutrients for continuous culture of the organism and all the sugars are utilised to some extent. Ethanol yields increase at lower pH and the product ratios are consistent with metabolism via the pentose phosphate pathway, glycolysis and PFL plus PDH (see Fig. 1).

A TWO-STAGE AEROBIC/ANAEROBIC FERMENTATION.

The property of quantitative conversion of sugars to ethanol without growth is a significant potential advantage of the new strain. It can be maximised by further manipulation of physiological constraints, as illustrated above, or by selection of further mutations. Figure 1 shows that cells lacking acetate kinase or acetyl CoA-phosphotransacetylase cannot produce acetate. Since acetate secretion is essential to maintain anaerobic flux through PFL, only the PDH-pathway remains open for pyruvate metabolism resulting in ethanol + $CO_2$. Such cells may not grow anaerobically but can be produced aerobically and used anaerobically to convert sugars to ethanol catalytically.

Moreover we have seen that mutations that increase intracellular pyruvate dehydrogenase activity will increase ethanol productivity, since PDH appears to limit energy flux. Such mutations will be selected, either spontaneously or after mutagenesis, by growth in continuous culture or on plates under conditions in which intracellular acetate and formate accumulation occur, i.e. on sugars at low pH + added acetate and formate. Alternatively additional copies of the PDH-genes can be introduced by genetic engineering protocols.

Since maximum ethanol productivity is associated with cessation of growth, conventional anaerobic batch cultures are unsuitable for ethanol production by such strains. Batch production may be achieved by using a large inoculum of cells grown aerobically into the anaerobic reactor, or by conducting batch fermentations under conditions of partial anaerobiosis, where the total biomass will depend on the level of oxygen supplied.

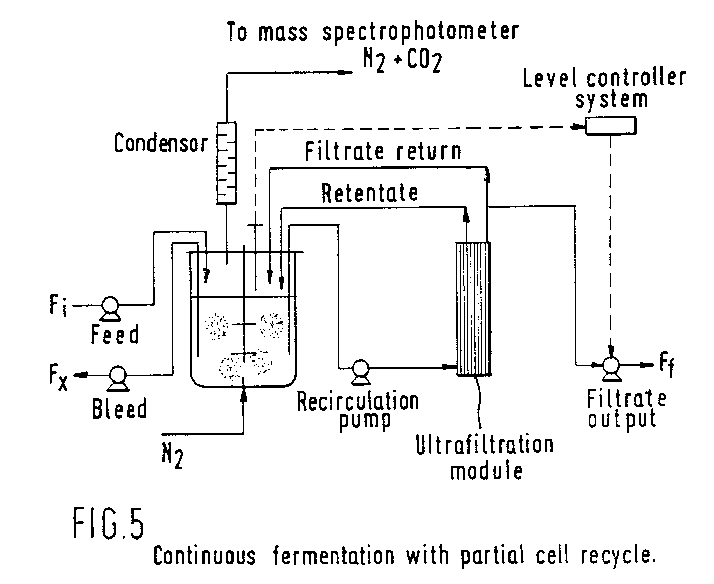

Moreover an indefinite continuous process catalysed by non-growing cells is clearly impossible; a minimum uptake of sugar (maintenance coefficient, $m_3$) is needed to maintain cell viability. We have seen that this can be achieved in a single-stage anaerobic reactor with partial cell recycle such as that illustrated in Fig. 5 without recycle or bleed; the system operates as a conventional single-stage continuous culture through the level controller system. When this is blocked and recycle begins, biomass levels rise to a maximum dictated by the maintenance coefficient. Thereafter all substrate is converted to products. This is clearly advantageous for production purposes, but will in practice lead to steadily decreasing (m) reactive productivity. However if a small bleed is taken from the reactor ($F_x$), steady-state growth occurs at a rate $\mu$ = $F_x/V$ (where V = reactor volume). This can be minimised to balance declining reactor productivity. In the figure, sugars and nutrients are pumped in at a rate $F_i$. A constant bleed $F_x$,($F_x \ll F_i$) determines the cell growth rate $\mu$ = $F_x/V$, (V = fermentor volume). The remaining broth is recirculated through a hollow fiber ultrafiltration membrane, operated at its maximum capacity. The filtrate output $F_f$ is controlled by a level controller system; the excess filtrate is returned to the fermentor.

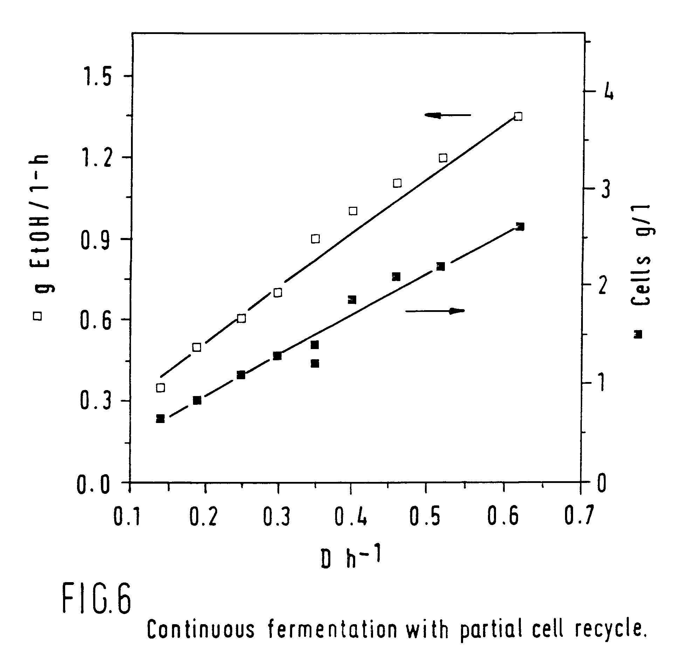

Figure 6 shows the results of a model system with strain LLD-15 on 1% sucrose/BST AM at 70°C, pH 7. The figure shows the relation between volumetric ethanol productivity, cell concentration and total dilution rate, D = $F_1/V$. $S_o$ = 1%, T 70°C, 400 rpm, pH 7.0 Bacillus stearothermophilus LLD-15. Cell growth rate $\mu$ = $F_x/V = 0.1h^{-1}$. The growth rate was kept constant at 0.1 $h^{-1}$, by fixing the bleed rate $F_x$. The overall dilution rate D was increased by increasing the sugars and nutrients feed rate $F_i$. Sucrose consumption (not shown) was always above 97% indicating a high stability of the system. The volumetric ethanol productivities were significantly higher than conventional single stage continuous fermentations (i.e. 0.6 g ethanol/l-h). This was primarily due to the proportional increase in cell density achieved at high dilution rates.

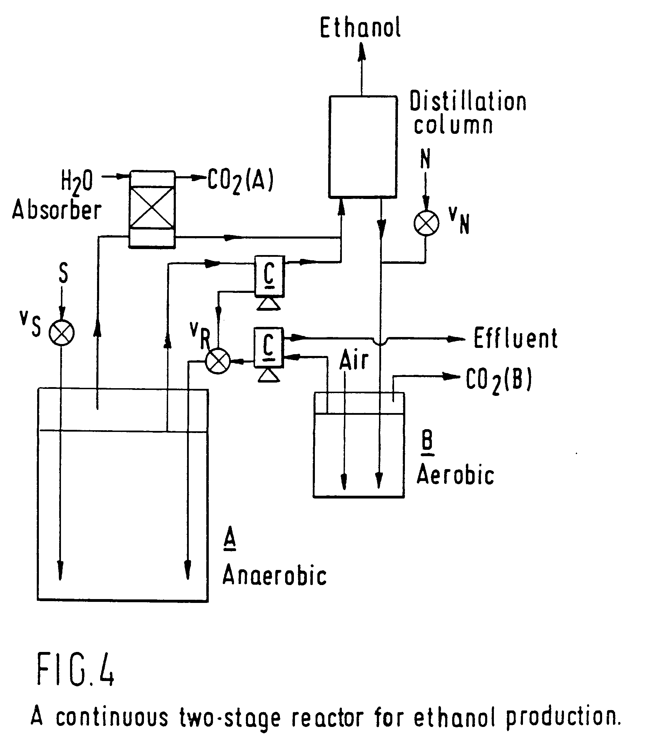

Such reactor systems are feasible for ethanol production by these strains, but we have seen that the special properties of a facultative anaerobe can be maximised for ethanol production in the novel reactor configuration illustrated in Fig. 4. In summary, sugars are pumped into the anaerobic reactor A at rate $V_2$. Vapour phase ethanol is separated from $CO_2$ by water absorption. A portion of spent cells is removed by centrifugation (C) and ethanol distilled from the effluent stream. The residual sugars and ethanol are supplemented with nutrients (N) at rate $V_n$ and used to create catalytic biomass aerobically (B). The resulting cells are returned to reactor A after centrifugation. In more detail sugars such as cane-juice, molasses, straw-hydrolysate, etc., are fed at rate $V_S$ into an anaerobic reactor supplied by cells at rate $V_R$. For the purpose of illustration the reactor A is a simple stirred tank (volume $V_A$) in which temperature and pH are controlled to maximise ethanol yield and productivity. Ethanol in the vapour phase is separated from $CO_2$ by absorption with water before continuous distillation. However one of the major advantages of a thermophilic fermentation is that as the boiling point of aqueous ethanol is approached it can be removed continuously and economically from the aqueous phase to remove ethanol inhibition of growth and/or productivity (in the case of strain LLD-15, growth ceases above 4% (w/v) ethanol at 60°C). This allows the use of higher concentrations of sugars as feedstock, such as molasses. Hence the anaerobic reactor may with advantage be one that maximises rate of ethanol removal into vapour phase such as vacuum fermentation, sparging with recycled $CO_2$ or continuous recycling through a vacuum flash evaporator.

Most of the cells in the effluent from A are concentrated by continuous centrifugation and recycled. Then ethanol is removed from the supernatant by continuous distillation. The stream entering the aerobic reactor B will contain spent cells (or spores), residual ethanol, unutilised sugars and by-products such as acetate and formate. Most of these can serve as aerobic substrates for strain LLD-15. Hence the stream is supplemented with necessary nutrients to allow maximum conversion of these waste carbon sources to biomass.

That biomass is concentrated by centrifugation and returned to the anaerobic reactor (volume $V_B$). There is a problem in that a lag phase may be observed before aerobic cells become adapted to anaerobic metabolism. Hence it may be advantageous to operate reactor B under oxygen limitation or to interpose an intermediate 'anaerobic adaptation' reactor fed with low sugars at optimum growth pH before the cells return to the catalytic stage.

The process variables in such a reactor configuration are complex, but the system has a redeeming feature. Optimal ethanol yield for any given feed composition and rate ($V_S$) occurs when anaerobic $CO_2$ (= ethanol) production is maximal and aerobic $CO_2$ (assuming complete sugars oxidation) is minimal. Optimal productivity is given by maximising $V_S$. Hence by using $CO_2$ sensors to control the pump rates, pH and

EP 0 370 023 B1

temperature in each vessel, the system lends itself to self-optimisation. This is a considerable advantage in minimising pilot plant development work with any particular substrate and an even greater advantage at plant scale in dealing with feedstocks of variable composition.

SUMMARY

In its preferred form, now summarised but without derogation from the claims, the present invention uses mutants of an extremely thermophilic facultative anaerobe such as the novel Bacillus stearothermophilus strain LLD-R (NCIB 12403) capable of rapid aerobic and anaerobic growth and/or metabolism above 70°C with a wide range of sugars including pentoses and cellobiose arising from hydrolysis of lignocellulose. The mutants, such as strain Bacillus thermophilus LLD-15 (NCIB 12428), are desirably selected so as to switch anaerobic pathways predominantly towards ethanol production. These are the deposited strains referred to herein.

Strain LLD-R grows rapidly on a wide range of sugars up to 75°C but the major anaerobic product is L-lactate. The mutant strain LLD-15 grows equally rapidly via two major energy pathways : the pyruvate - formate lyase (PFL) pathway yielding 1 mole ethanol, 1 acetate and 2 formate/mole glucose residue, and a previously unrecognised pyruvate dehydrogenase (PDH) pathway yielding 2 ethanol + 2 $CO_2$/mole glucose.

The metabolic flux in strain LLD-15 can be directed through the PDH-pathway by manipulation of physiological conditions, in particular a build-up of pyruvate caused by growth at acid pH or by the presence of acetate and formate in the medium. Higher temperatures also favour the PDH-pathway. The cells may not grow under such conditions but continue to convert sugars to ethanol. Alternatively the PDH-flux can be increased by further mutations; for example mutations which suppress acetate production. Other desirable mutations are those which increase total anaerobic pyruvate dehydrogenase activity, since this is rate-limiting for ethanol productivity.

Such strains are optimal for a two-stage fermentation in which catalytic biomass is first grown in an aerobic seed stage and subsequently used anaerobically in an ethanol production stage without growth. This can be achieved in a single-stage batch or fed-batch reactor, with continuing ethanol removal in the vapour phase to allow use of concentrated sugar feedstocks. Conventional feedstocks such as glucose, sucrose or maltose may be used, but also sugars arising from hydrolysis of lignocellulosic wastes including pentoses and cellobiose.

The strains are not very suitable for conventional single-stage continuous cultures but may be used to advantage in a single stage system with partial cell recycle or in a two-stage continuous system in which sugars are fed to an anaerobic catalytic reactor with continuing ethanol removal in the vapour. The remaining ethanol is strapped from the aqueous effluent from this reactor and the residual carbon sources are utilised to create new catalytic biomass in an aerobic biomass stage. Thereby effectively all of the potential substrates in the feedstocks are utilised either anaerobically or aerobically. Moreover this system lends itself to automated self-optimisation for ethanol production by maximising anaerobically-produced $CO_2$ (equivalent to biomass production). This can be a particular advantage when using mixed and variable feedstocks.

REFERENCES

1. Hartley, B.S. et. al. (1983) In 'Biotech 83' Online Publications Ltd., Northwood, U.K., p. 895.
2. Hartley, B.S. and Shama, G. (1987) In 'Utilisation of Cellulosic Wastes' (eds. Hartley, B.S., Broda, P.M.A., and Senior, P.), The Royal Society, London.
3. Payton, M.A. and Hartley, B.S. (1985) FEMS Microbiol. Lett. 26, 333.
4. Ragg, P.L. and Fields, P.R. In 'Utilisation of Lignocellulosic Wastes' (Eds. Hartley, B.S., Broda P.M.A. and Senior, P.), The Royal Society, London.

**Claims**

**1.** Production of ethanol by a process in which a strain of Bacillus stearothermophilus or other thermophilic, facultatively anaerobic bacterium is selected for the characteristics of fermenting sugars both aerobically and anaerobically and of being active in anaerobic fermentation at 70°C or above and

(i) anaerobic fermentation is carried out with continuing removal of ethanol at 70°C or above

(ii) the fermentative activity of the bacterium is maintained by withdrawing a proportion of the anaerobic fermentation medium on a continuing basis preferably with removal of ethanol and

10

allowing the bacteria therein to multiply aerobically, using residual sugars or metabolites thereof present in the medium, before being returned to the anaerobic fermentation.

2. Production of ethanol according to claim 1, wherein the bacterium is selected for the further characteristic of entering, after an aerobic or anaerobic growth phase, a non-multiplying but metabolically active anaerobic phase wherein essentially only ethanol is produced, and the anaerobic fermentation is carried out substantially in that phase.

3. The process according to claim 1, wherein anaerobic and aerobic $CO_2$ production is monitored and the anaerobic $CO_2$ to aerobic $CO_2$ ratio is maximized for each of the process conditions employed.

4. Production of ethanol according to any preceding claim, wherein, specifically, the bacterium is selected as essentially lacking NAD-linked lactate dehydrogenase activity.

5. Production of ethanol according to any preceding claim, wherein, specifically, the bacterium is selected as producing the ethanol essentially by pyruvate dehydrogenase pathway activity.

6. Production of ethanol acccording to claim 5, wherein, specifically, the bacterium is selected as one in which, during the fermentation, pyruvate-formate lyase pathway flux is suppressed by mutation or by conditions causing intracellular accumulation of metabolites.

7. Production of ethanol according to any preceding claim, wherein the sugars include pentoses or cellobiose.

8. Production of ethanol according to any preceding claim, specifically by a strain of Bacillus stearothermophilus.

9. Production of ethanol according to claim 8 wherein, specifically B. stearothermophilus LLD-R (NCIB 12403) or B. stearothermophilus LLD-15 (NCIB 12428) or variants thereof having the same function as the parent strain are used.

10. Bacillus stearothermophilus LLD-R (NCIB 12403) and Bacillus stearothermophilus LLD-15 (NCIB 12428) and ethanol producing variants thereof.

**Patentansprüche**

1. Herstellung von Ethanol durch ein Verfahren, in dem ein Stamm von Bacillus stearothermophilus oder ein anderes thermophiles, fakultativ anaerobes Bakterium für die Merkmale, Zucker sowohl aerob als auch anaerob zu fermentieren und in anaerober Fermentation bei 70°C oder darüber aktiv zu sein, ausgewählt wird, und
(i) anaerobe Fermentation mit fortlaufender Entfernung von Ethanol bei 70°C oder darüber durchgeführt wird
(ii) die fermentative Aktivität des Bakteriums aufrechterhalten wird durch Abziehen eines Anteils des anaeroben Fermentationsmediums auf einer fortlaufenden Basis vorzugsweise mit Entfernen von Ethanol und Ermöglichen aerober Vermehrung der Bakterien darin unter Verwendung von im Medium anwesenden Restzuckern oder deren Metaboliten, vor der Rückführung zur anaeroben Fermentation.

2. Herstellung von Ethanol gemäß Anspruch 1, bei der das Bakterium ausgewählt wird für das weitere Merkmal des Eintretens, nach einer aeroben oder anaeroben Wachstumsphase, in eine nicht vermehrende, aber metabolisch aktive anaerobe Phase, in der im wesentlichen nur Ethanol erzeugt wird, und die anaerobe Fermentation im wesentlichen in dieser Phase durchgeführt wird.

3. Verfahren gemäß Anspruch 1, bei dem anaerobe und aerobe $CO_2$-Erzeugung überwacht und das Verhältnis von anaerobem $CO_2$ zu aerobem $CO_2$ für jede der angewandten Verfahrensbedingungen maximiert wird.

**4.** Herstellung von Ethanol gemäß irgend einem vorhergehenden Anspruch, bei der speziell das Bakterium ausgewählt wird, dem im wesentlichen NAD-gebundene Lactatdehydrogenase-Aktivität fehlt.

**5.** Herstellung von Ethanol gemäß irgend einem vorhergehenden Anspruch, bei der speziell das Bakterium ausgewählt wird, das das Ethanol im wesentlichen durch Pyrvvatdehydrogenase-Weg-Aktivität herstellt.

**6.** Herstellung von Ethanol gemäß Anspruch 5, bei der speziell das Bakterium als ein solches ausgewählt wird, in dem während der Fermentation der Pyruvat-formiat-Lyase-Weg-Fluß durch Mutation oder durch Bedingungen die intrazelluläre Akkumulation von Metaboliten verursachen, unterdrückt wird.

**7.** Herstellung von Ethanol gemäß irgend einem vorhergehenden Anspruch, bei der die Zucker Pentosen oder Cellobiosen einschließen.

**8.** Herstellung von Ethanol gemäß irgend einem vorhergehenden Anspruch, speziell durch einen Stamm von Bacillus stearothermophilus.

**9.** Herstellung von Ethanol gemäß Anspruch 8, bei der speziell B. stearothermophilus LLD-R (NCIB 12403) oder B. stearothermophilus LLD-15 (NCIB 12428) oder Varianten davon, die dieselbe Funktion wie der Ausgangsstamm besitzen, verwendet werden.

**10.** Bacillus stearothermophilus LLD-R (NCIB 12403) oder Bacillus stearothermophilus LLD-15 (NCIB 12428) und Ethanol-herstellende Varianten davon.

**Revendications**

**1.** Production d'éthanol par un procédé dans lequel une souche de Bacillus stearothermophilus ou d'une autre bactérie thermophile, facultativement anaérobie, est choisie pour ses caractéristiques de fermenter des sucres, à la fois par voie aérobie et par voie anaérobie, et d'être active en fermentation anaérobie à 70°c ou au-dessus, et

(i) une fermentation anaérobie est effectuée avec élimination en continu de l'éthanol à 70°C ou au-dessus,

(ii) l'activité de fermentation de la bactérie est maintenue en éliminant une proportion du milieu de fermentation anaérobie, sur une base continue, de préférence avec élimination de l'éthanol et en permettant aux bactéries présentes de se multiplier par voie aérobie, en utilisant ses sucres ou métabolites résiduels présents dans le milieu, avant d'être retournée à la fermentation anaérobie.

**2.** Production d'éthanol selon la revendication 1, dans laquelle la bactérie est choisie pour la caractéristique additionnelle de parvenir, après une phase de croissance aérobie ou anaérobie, à une phase anaérobie de non-multiplication mais métaboliquement active, dans laquelle pratiquement seul de l'éthanol est produit, et la fermentation anaérobie est effectuée essentiellement dans cette phase.

**3.** Procédé selon la revendication 1, dans lequel on contrôle une production anaérobie et aérobie de $CO_2$ et le rapport du $CO_2$ anaérobie au $CO_2$ aérobie est maximisé pour chacune des conditions de procédé utilisées.

**4.** Production d'éthanol selon l'une des revendications précédentes, dans lequel on choisit spécifiquement la bactérie comme étant dépourvue essentiellement d'une activité de lactate déhydrogénase liée au NAD.

**5.** Production d'éthanol selon l'une des revendications précédentes, dans laquelle on choisit spécifiquement la bactérie comme produisant l'éthanol essentiellement par une activité par vote de pyruvate déshydrogénase.

**6.** Production d'éthanol selon la revendication 5, dans laquelle on choisit spécifiquement la bactérie comme étant l'une dans laquelle, au cours de la fermentation, le flux de pyruvate-formate lyase est supprimé par mutation ou par des conditions provoquant une accumulation intracellulaire de métabolites.

12

7. Production d'éthanol selon l'une des revendications précédentes, dans laquelle les sucres comprennent des pentoses ou de la cellobiose.

8. Production d'éthanol selon l'une des revendications précédentes, spécifiquement par une souche de Bacillus stéarothermophilus.

9. Production d'éthanol selon la revendication 8, dans laquelle on utilise spécifiquement B. stéarothermophilus LLD-R (NCIB 12403) ou B. stearothermophilus LLD-15 (NCIB 12428) ou ses variantes ayant la même fonction que la souche mère.

10. Bacillus stearothermophilus LLD-R (NCIB 12403) et Bacillus stearothermophilus LLD-15 (NCIB 12428) et ses variantes produisant de l'éthanol.

# FIG.1

Anaerobic pathways and theoretical yields in
<u>Bacillus stearothermophilus</u> strain LLD-15.

FIG.2 Steady state values in continuous cultures at pH7, D=0.2hr$^{-1}$ on sucrose 10g./1. anaerobically or 5g./1. aerobically (dotted line), 0.5% tryptone, 0.25% yeast extract:
a) Biomass: (●) strain NCA 1503, (◨) strain LLD-R, (○) strain LLD-15.
b) Products with strain LLD-15.

FIG.3

Continuous cultures of strain LLD-15 on various concentrations of wheat-straw hydrolysate (Ragg and Fields, 1986) at 70°C. D = 0.2hr$^{-1}$, ph7.

FIG.4

A continuous two-stage reactor for ethanol production.

**FIG.5**

Continuous fermentation with partial cell recycle.

FIG.6

Continuous fermentation with partial cell recycle.